# EUROPEAN PATENT SPECIFICATION

(11) **EP 1 589 941 B1**
(45) Date of publication and mention of the grant of the patent: **27.02.2019**
(21) Application number: 04706387.0
(22) Date of filing: 29.01.2004
(51) Int. Cl.: A61K 9/06, A61K 33/30, A61P 31/22, A61P 17/16, A61K 8/27

(54) **COMPOSITION FOR PREVENTIVE TREATMENT OF COLD SORES**
ZUSAMMENSETZUNGEN FÜR DIE PRÄVENTIVE BEHANDLUNG VON KÄLTESCHÄDEN
PREPARATION POUR LE TRAITEMENT PREVENTIF DE L'HERPES LABIAL

(30) Priority: 30.01.2003 NL 1022532
(43) Date of publication of application: 02.11.2005
(73) Proprietor: Laboratory PM B.V., 1184 VW Ouderkerk a/d Amstel (NL)
(72) Inventor: HARTMAN, Gerrit, Egbert, NL1243 KH 's-Graveland (NL)
(74) Representative: Nederlandsch Octrooibureau
(86) International application number: PCT/NL2004/000069
(87) International publication number: WO 2004/066971

(56) References cited:
- WO-A-99/47109
- FR-A- 2 824 474
- US-B1- 6 475 526
- DATABASE MEDLINE [Online] US NATIONAL LIBRARY OF MEDICINE (NLM), BETHESDA, MD, US; R C LUNDEEN ET AL.: "Sunscreen protection for lip mucosa: a review and update" XP002255573 Database accession no. nlm3877087 & THE JOURNAL OF THE AMERICAN DENTAL ASSOCIATION, vol. 111, no. 4, October 1985 (1985-10), pages 617-621,
- VAN DER MOLEN R G ET AL: "Ultraviolet-B radiation induces modulation of antigen presentation of herpes simplex virus by human epidermal cells." HUMAN IMMUNOLOGY. JUN 2001, vol. 62, no. 6, June 2001 (2001-06), pages 589-597, XP002282094 ISSN: 0198-8859

## Description

The present invention relates to a composition for preventive, topical treatment of the symptoms associated with the productive phase of infections by the Herpes Simplex Virus (HSV), such as fever blisters, including cold sores.

The Herpes Simplex Virus is a virus which infects nerve cells in mammals. In humans two types of HSV occur, HSV-1 mainly infects the nerve endings in the face, particularly the area around nose and mouth, while type 2 mainly infects the nerve endings in the anogenital area. The molecular reason for this tissue preference is unknown; nor is this an absolute preference, both HSV-1 and HSV-2 can be found in the facial area and in the anogenital area. Of adults in the US and Europe, 80% is carrier of HSV-1 and 20% is carrier of HSV-2.

The productive phase of the infection of the HSV manifests itself on the skin with one or more of the following symptoms: blisters, itching, a burning sensation and scab formation. If they occur in the face, these symptoms are referred to in common usage as 'fever blisters' or a 'cold sore' (herpes labialis) if the symptoms occur on the lips. These symptoms can affect the appearance of the face. Due to this, and the itching, a general feeling of discomfort can result in the patient with fever blisters.

In the infection the virus DNA integrates in the host DNA, were it remains for life as "provirus". The productive phase of the virus can be induced by the influence of environmental factors and host factors. Environmental factors which can herald a productive phase of HSV are sunlight (UV wavelength) and infectious diseases. Host factors which play a part in the suppression of the productive phase are the immune system and the oxidation/antioxidation status. The recurrence of a productive phase is here often accompanied by a recurrence of one or more of the above described symptoms and the sensation of discomfort associated therewith.

The infection by both types of HSV is identical: the productive phase manifests itself on the skin with a burning and painful sensation with forming of blisters for 2-4 days (on average 3 days), followed by scab formation and wound healing for 4-7 days.

An HSV infection is generally harmless in adults with a fair to good all-round state of health, but can be life-threatening in newborn babies; probably because babies have not yet been able to build up an immune reaction to HSV.

It will be sufficiently apparent from the foregoing that an effective prevention of the productive phase of HSV infections is important, since fever blisters (including cold sores) can hereby be prevented.

Prevention of the productive phase of HSV infections and treatment of the symptoms of the productive phase, i.e. cold sores, with topical agents is known from the prior art.

Kneist et al. (Arzneimittelforschung 1995; 45(5):624-6) thus describe clinical data of the effectiveness of zinc sulphate in the symptomatic treatment of Herpes labialis recidivans.

Brody et al. (Br J Dermatol 1981; 104(2):191-4) describe the effect of topical administering of zinc sulphate on recurrent Herpes Simplex infections.

Kumel et al. (J Gen Virol 1990; 71 (Pt 12):2989-97) describe the molecular mechanisms possibly forming the basis of the inhibition of HSV infections by zinc ions. In their opinion this inhibiting action is linked to an inhibiting effect of the zinc ions on the function of a glycoprotein that plays a part in the penetration of the virion in the host cell.

International patent application WO 99/47109 describes a sunscreen formulation comprising a) organic or b) inorganic light protection filters or a combination of a) and b) light protection filters and wherein the light protection filters absorb in the UVA, UVB, IR and visible region of the light spectrum. Titanium oxide and zinkc oxide are mentioned as examples of inorganic light protection filters.

American patent US 6475526 describes the topical use of a composition comprising a combination of a zinc compound and an antioxidant in the symptomatic treatment of HSV infections.

In addition to the topical use of zinc ions, the effectiveness of using a chemical sun filter on the skin has been researched by Rooney et al. in relation to recurrent herpes labialis (Lancet 1991 7;338(8780):141922). The authors conclude from their research that use of a (chemical) sun filter can be effective in the prevention of sunlight-induced recurrent infections.

The prior art thus describes on the one hand two distinct types of topical agents, zinc sulphate or a chemical sun filter, and on the other hand three distinct methods of employing these agents: (a) preventing cold sores by applying a chemical sun filter before the skin is exposed to sunlight; (b) applying zinc sulphate after the first signs of a cold sore have manifested themselves and continuing to apply zinc sulphate until the cold sore has disappeared; and (c) the same as method 'b' but with the difference that, after the cold sore has disappeared, zinc sulphate is applied for life, periodically and according to a fixed regime, in order to prevent the cold sore from returning.

The agents and methods for their use mentioned in the prior art are insufficiently effective to prevent cold sores. It has thus been found that in many cases the application of only a UV-filter produces an insufficiently protective effect, probably because sunlight is only one of the factors which can induce a productive phase of HSV. It has further been found that in many cases application of zinc sulphate alone produces an insufficiently protective effect in the case of exposure to sunlight, probably because zinc sulphate (in the concentration used) cannot prevent the productive phase of the Herpes virus from being induced at a determined quantity of sunlight, or because other factors which can initiate the productive phase can occur simultaneously with sunlight. Nor are the methods effective, since lifelong application of a medication is generally considered undesirable. This is moreover impractical and expensive. Against this background, applicant has been seeking more effective agents for preventing the productive phase of HSV infections and the fever blisters (including cold sores) associated therewith.

Applicant has found that the topical administering in the face of a combination of zinc ions and a physical UV filter in the form of zinc oxide and/or titanium oxide, at the moment when the person in question is (possibly) at an increased risk of developing a fever blister, or certainly does not wish to develop a fever blister, is more effective in preventing fever blisters than that which is described in the prior art.

The invention therefore relates to a composition for use in the preventive treatment of fever blisters, including recurring fever blisters, wherein the composition comprises:
- 4-20% (w/w) of zinc oxide and/or titanium dioxide;
- in respect of the zinc ions, 0.1-2.0% (w/w) of a water-soluble zinc salt with antiviral action, said zinc salt being selected from the group consisting of zinc sulphate, zinc chloride, zinc acetate, zinc citrate, zinc nitrate, zinc tartrate, zinc maleate, zinc lactate, zinc amino acetate, zinc aspartate, zinc glutamate, zinc propionate, zinc gluconate, zinc butyrate, zinc formiate, zinc glyceride, zinc glycolate, and their hydrates; and
- an additive suitable for topical application;
wherein the composition is preventively applied topically to parts of the face which can be affected by fever blisters.

The compositions according to the invention are effective in the prevention of fever blister due to their preventive effect on the development of the productive phase of an HSV infection, including a recurring HSV infection. Due to the link between fever blisters and the productive phase of an HSV infection, fever blisters must be understood in the context of the present invention to also mean the productive phase of an HSV infection. Fever blisters are also understood to include a cold sore.

The composition according to the invention can be applied from the moment that there is (possibly) an increased risk of developing fever blisters, up until several days after the (possibly) increased risks have disappeared or are at least considerably reduced. An increased risk can result from exposure of the face to sunlight (UV radiation) but also from, among others, fatigue and/or stress and/or illness. Reference is intentionally made here to a '(possibly) increased risk', since fatigue and stress cannot usually be determined objectively. If a person who regularly has fever blisters wishes to be certain that he/she does not have fever blisters at a specific moment, he/she can also begin applying the composition according to the invention several days before this specific moment.

The composition is generally applied to the face, such as on the parts around the mouth and nose, for instance on the lips, in the case of an anticipated increased exposure to UV radiation or in the case of stress or fatigue. Application of the composition can be stopped several days after the increased risk has disappeared. In addition, the composition as described above can be applied to the described parts of the face several days prior to the moment at which fever blisters are absolutely undesired. The moment at which the fever blisters are undesired may be a period of a number of days. The composition according to the invention is then applied to the parts of the face which can be affected by fever blisters for several days before this period of time and up to the last day of this period of time. In the context of the present invention, several days are here 1-5 days, for instance 1, 2, 3 or 4 days.

During the period of its use the composition according to the invention can be applied as often as necessary to the parts of the face which can be infected by HSV. The composition is preferably applied to the parts of the face where fever blisters have already occurred before. It is generally sufficient to apply the composition 1-3 times a day, and the composition is preferably applied 2 or 3 times a day. In the case of an increased exposure to UV radiation, it is however recommended to apply the composition to the skin at intervals of 2-4 hours during this increased exposure.

In the context of the present invention, an increased exposure to UV radiation is understood to mean a prolonged period of exposure as well as an increased radiation intensity. The UV radiation can originate from a natural source, such as the sun, or from an artificial source.

The compositions according to the invention comprise a physical UV filter in the form of zinc oxide and/or titanium oxide. The advantage of using such a physical UV filter over a chemical filter is the long-lasting action, since in contrast to a chemical filter a physical filter is not converted by the radiation. In addition, there occur no, or at least fewer, hypersensitivity reactions to the physical UV filters.

Hypersensitivity reactions can bring about inflammation reactions which, when they occur in the face, can also be accompanied by an effect on the appearance of the face.

The physical UV filter comprises zinc oxide or titanium dioxide or a combination hereof as active constituent. The quantity and form of the physical UV filter is such that a composition is obtained with a sun protection factor (SPF) greater than 12, preferably greater than 15, most preferably greater than 20. In addition, it is recommended that the physical UV filter does not essentially contribute toward the colour of the composition according to the invention. The quantity and form of the physical UV filter is preferably such that, despite its presence, an essentially transparent composition can be manufactured. The physical filter is preferably a finely or ultra-finely distributed material, such as a micronized material, for instance micronized zinc oxide or micronized titanium dioxide, since this reduces the colour effect. The average size of the particles is herein smaller than about 5 µm, for instance smaller than about 2 µm, preferably smaller than about 1 µm, more preferably smaller than about 0.05 µm, such as 0.02 µm. Suitable quantities of the physical UV filter (zinc oxide and/or titanium dioxide) lie between 8-12% (w/w), such as 10% (w/w) relative to the total weight of the composition. As physical UV filter a combination of zinc oxide and titanium dioxide is preferably applied in a ratio of 5:1 to 2:1, preferably 3:1 to 2:1, most preferably 3:1. In order to obtain a good UV-screening action, it is important to suspend the particles of the physical UV filter uniformly in the composition.

In addition to zinc oxide and/or titanium oxide, the composition according to the present invention also comprises as active constituent a water-soluble zinc salt with antiviral action, said zinc salt being selected from the group consisting of zinc sulphate, zinc chloride, zinc acetate, zinc citrate, zinc nitrate, zinc tartrate, zinc maleate, zinc lactate, zinc amino acetate, zinc aspartate, zinc glutamate, zinc propionate, zinc gluconate, zinc butyrate, zinc formiate, zinc glyceride, zinc glycolate, and their hydrates. Zinc sulphate is recommended.

In respect of the zinc ions, the quantity of the water-soluble zinc salt with antiviral action in the composition according to the invention can be between 0.1-1% (w/w), more preferably 0.5-1% (w/w) relative to the total weight of the composition. A water-soluble zinc salt will be present substantially only in a water phase or a water-miscible phase. In respect of the zinc ions the quantity of the water-soluble zinc salt can therefore also be expressed relative to the quantity of water (water-miscible phase) and amounts in that case to 0.07-5% (w/w), preferably 0.17-2% (w/w) and more preferably 0.275-1.8% (w/w).

In addition to the active constituents, the composition according to the present invention comprises at least one additive suitable for topical use. The additive is preferably a carrier and/or diluent such as a fat or an oil, or a combination hereof.

Fats can be selected from one or more of lanette cream, cetomacrogol cream, wool fat, wool wax alcohols, preferably one or more of (white) vaseline and liquid paraffin. The proportion of fat in the composition according to the invention can lie between 5-30% (w/w), such as 10-20% (w/w), preferably 15-20% (w/w).

Oils can be selected from one or more of arachide oil, oleic acid, canola oil, corn oil, cotton-seed oil, ethyl oleate, isopropyl palmitate, sesame oil, soya oil and cetiol V. The proportion of oil in the composition can lie between 10 and 30% (w/w), such as 15-20% (w/w).

The composition according to the invention will further contain water or another polar solvent, such as propylene glycol, for the purpose of dissolving the water-soluble zinc salt with antiviral action and making free zinc ions available. The use of water as polar solvent is recommended, since it is readily available and is cosmetically well tolerated. The water or other polar solvent is preferably added in a quantity of 40-70% (w/w), such as 50-60% (w/w), preferably 55% (w/w).

If water is added in addition to an oil and/or fat carrier, an emulsion, more preferably a stable emulsion, is preferably formed from the water phase and oil/fat phase. Such an emulsion is preferably an oil (fat) in water (O/W) emulsion, wherein the water phase is the continuous (outer) phase and the oil (fat) phase is the discontinuous (enclosed) phase. Because in an O/W emulsion the water phase is included as outer phase, the water-soluble zinc salt is readily available for absorption by the skin when the composition according to the invention is applied thereto. In a water in oil (fat) (W/O) emulsion, the water phase is enclosed in the oil phase, whereby the water-soluble zinc salt is less readily available for absorption by the skin.

In order to emulsify the water and oil phase, one or more emulsifiers can be added to the composition according to the invention. A stable emulsion is preferably formed using the emulsifier. A stable emulsion is here understood to mean an emulsion wherein, for an extended period of time such as longer than a week, preferably longer than a month, more preferably longer than 6 months, most preferably longer than a year, no separation of the oil (fat) phase and the water phase occurs and/or no transposition from an O/W emulsion to a W/O emulsion occurs.

It has been found that in particular a stable o/w emulsion is an exceptionally suitable presentation form of the composition according to the invention. As stated, in such an o/w emulsion the water-soluble zinc salt is readily available for absorption by the skin. In addition, the physical UV-filter in the form of zinc oxide and/or titanium oxide can be incorporated in the oil (fat) phase. This is particularly recommended if zinc oxide and/or titanium dioxide are applied as physical UV filter because of the hydrophobic properties of these substances. In a particularly preferred embodiment, the composition according to the invention in respect of the zinc ions, comprises, relative to the total composition, a quantity of zinc salt of 0.5-1% (w/w); 15-25%, preferably 20% of a fat, preferably a combination of white vaseline and liquid paraffin; 5-15%, preferably about 10% of a physical UV filter, preferably a 1:4, such as 1:3, combination of zinc oxide and titanium dioxide; 50-60%, preferably 52-58%, most preferably 55% (w/w) water; and an emulsifier which is suitable for forming an oil (fat) in water emulsion.

A suitable emulsifier can be selected from the group of cetyl and stearyl alcohol, Eumilgin B2 (cetomacrogol 1000), stearic acid, lanolin, lanolin alcohols, light and heavy mineral oils, petrolatum, beeswax, paraffin wax, tween derivatives, span derivatives or combinations thereof. The emulsifier can be applied in a quantity of 2-20% (w/w), such as 5-15% (w/w), preferably 7-12% (w/w). A combination of cetyl and stearyl alcohol and Eumilgin B2 (cetomacrogol 1000) is preferably used to emulsify the composition. This combination of cetyl and stearyl alcohol and Eumilgin B2 (cetomacrogol 1000) can be applied in a ratio of 1:5 to 5:1, preferably a ratio of 3:1. The combined quantity of cetyl and stearyl alcohol and Eumilgin B2 (cetomacrogol 1000) is for instance 7-12% (w/w), such as 10.5% (w/w).

One or more detergents, such as NaOH or laurenth sulphate, can further be added as additive. Addition of NaOH and/or laurenth sulphate can help bring about better penetration of the skin by the zinc ions. Suitable applicable quantities of these additives are known to the skilled person.

As stated above, it is important for a good UV-protective action to distribute the particles of zinc oxide and/or titanium oxide uniformly through the composition. If the composition is an emulsion, such as an oil (fat) in water (O/W) emulsion, the particles can be suspended in the oil (fat) phase. If the oil (fat) phase is uniformly distributed in the water phase, the particles of zinc oxide and/or titanium oxide are hereby also distributed uniformly in the composition.

The composition according to the invention can take the form of a cream or gel. The advantage of a cream or gel is that it can be used in hygienic manner. When a cream or gel is used, the chance of cross-infection of different parts of the body, particularly the face, is hereby relatively low compared to for instance the use of a stick. If used hygienically, a cream or gel can even be used by a number of people, without the occurrence of, or at least with a reduced chance of, infection between the people mutually.

The composition according to the invention is preferably not visible after application to the skin, since it is transparent in colour or has a colour which corresponds with the colour of lips and/or is quickly absorbed by the skin. In addition, it is advantageous if the composition is water-resistant. This prevents it losing its effect under the influence of water. This is particularly important if the composition is applied to the parts around the mouth. The water-resistant properties prevent the composition disappearing quickly from the lips through contact with saliva and/or with the tongue. Use of, among others, vaseline and/or liquid paraffin as additive gives the composition water-resistant properties.

The composition according to the invention is preferably suitable for cosmetic use.

The invention is further elucidated on the basis of the following exemplary embodiments. These exemplary embodiments must be deemed as practicable embodiments of the invention and in no way limit the scope of the invention.

### Example I:

A transparent, water-resistant cream was obtained by mixing the components below in the stated ratio, and tubes with a content of 30 ml were filled with the cream.

| | |
|---|---|
| zinc oxide, average particle size 0.1 µm | 10 g |
| zinc sulphate | 0.8 g |
| liquid paraffin | 44.6 g |
| lanette cream | 44.6 g |

### Example II:

A transparent, water-resistant cream was obtained by mixing the components below in the stated ratio, and tubes with a content of 50 ml were filled with the cream.

| | |
|---|---|
| zinc oxide, average particle size 0.05 µm | 16 g |
| zinc sulphate | 2 g |
| Vaseline | 36.4 g |
| lanette cream | 145.6 g |

### Example III:

A transparent, water-resistant cream was obtained by firstly (i) mixing the following substances of component A in the stated quantities while heating to 75-80%:
**A**

| | |
|---|---|
| Cetyl and stearyl alcohol | 8.0 g |
| Eumilgin B2 (cetomacrogol 1000) | 2.5 g |
| Isopropyl myristate | 1.5 g |
| White vaseline | 10.0 g |
| Liquid paraffin 110-230 mpas | 10.0 g |
| Sub-total | 32.0 g |

Then (ii) the substances of component B were suspended in the still warm mixture of component A.
**B**

| | |
|---|---|
| Pigment iron oxide red E 172 | 0.02 g |
| Zinc oxide | 7.5 g |
| Titanium dioxide | 2.5 g |
| Sub-total | 10.02 g |

(iii) The following substances of component C were mixed with or dissolved in water of about 80°C.

**C**

| | |
|---|---|
| Demineralized water (RO 80) | 55.0 g |
| Zinc sulphate | 1.0 g |
| Citric acid (for pH regulation) | 0.4 g |
| Allantoin | 1.5 g |
| Methyl parahydroxy benzoate (as preservative) | 0.05 g |
| Sub-total | 57.98 g |
| total | 100 g |

(iv) The mixture of step (iii) was mixed with the mixture of step (ii) and the whole was cooled to about 30°C.
(v) Add 0.03 g of Menthae piperitae aetheroleum.
(vi) Tubes with a content of 50 ml were filled with the cream with an SPF of 20.

## Claims

1. Composition for use in the preventive treatment of fever blisters, including recurring fever blisters, wherein the composition comprises:
• 4-20% (w/w) of zinc oxide and/or titanium dioxide;
• in respect of the zinc ions, 0.1-2.0% (w/w) of a water-soluble zinc salt with antiviral action, said zinc salt being selected from the group consisting of zinc sulphate, zinc chloride, zinc acetate, zinc citrate, zinc nitrate, zinc tartrate, zinc maleate, zinc lactate, zinc amino acetate, zinc aspartate, zinc glutamate, zinc propionate, zinc gluconate, zinc butyrate, zinc formiate, zinc glyceride, zinc glycolate, and their hydrates; and
• an additive suitable for topical application;
wherein the composition is preventively applied topically to parts of the face which can be affected by fever blisters.

2. Composition for the use as claimed in claim 1, wherein the composition is applied to the face from the moment that the user has an increased risk of developing fever blisters.

3. Composition for the use as claimed in claim 2, wherein the composition is applied up until several days, such as 1-5 days, after the increased risk has disappeared.

4. Composition for the use as claimed in claims 2-3, wherein the increased risk comprises an increased exposure to UV radiation, and the composition is applied at intervals of 2-4 hours during this increased exposure.

5. Composition for the use as claimed in claim 1, wherein the composition is applied to the face for several days, such as 1-5 days, before the day(s) on which the user wishes to certainly not have fever blisters.

6. Composition for the use as claimed in claim 5, wherein the composition is applied up to and including the day(s) on which the user wishes to certainly not have fever blisters.

7. Composition for the use as claimed in any of the claims 1-6, wherein the zinc salt is zinc sulphate.

8. Composition for the use as claimed in any of the claims 1-7, wherein the composition in respect of the zinc ions comprises a quantity of the zinc salt of not more than 0.5% (w/w).

9. Composition for the use as claimed in any one of claims 1-8, wherein the composition comprises between 8-12% (w/w), such as 10% (w/w) zinc oxide and/or titanium dioxide.

10. Composition for the use as claimed in any of the claims 1-9, in the form of a cream or gel.

11. Composition for the use as claimed in any of the claims 1-10, which further contains water in a quantity of 50-60% (w/w), preferably 52-58% (w/w), most preferably 55% (w/w), and wherein the additive is an oil/fat-like substance and the water phase and oil/fat phase form an oil/fat-in-water emulsion.

12. Composition for the use as claimed in claim 11, wherein the emulsion is a stable emulsion.

13. Composition for the use as claimed in any of the claims 1-12, wherein the additive is selected such that the composition is water-resistant.

14. Composition for the use as claimed in any of the claims 1-13, with a sun protection factor (SPF) of at least 12, preferably at least 15, and most preferably at least 20.

## Patentansprüche

1. Zusammensetzung zur Verwendung bei der präventiven Behandlung von Fieberbläschen, einschließlich wiederkehrender Fieberbläschen, wobei die Zusammensetzung umfasst:
• 4-20% (Gew./Gew.) Zinkoxid und/oder Titandioxid;
• in Bezug auf die Zink-Ionen 0,1 - 2,0 % (Gew./Gew.) eines wasserlöslichen Zinksalzes mit antiviraler Wirkung, wobei besagtes Zinksalz aus der Gruppe ausgewählt ist, welche aus Zinksulfat, Zinkchlorid, Zinkacetat, Zinkcitrat, Zinknitrat, Zinktartrat, Zinkmaleat, Zinklactat, Zinkaminoacetat, Zinkaspartat, Zinkglutamat, Zinkpropionat, Zinkgluconat, Zinkbutyrat, Zinkformiat, Zinkglycerid, Zinkglycolat und deren Hydraten besteht; und
• ein für die topische Anwendung geeignetes Additiv;
wobei die Zusammensetzung präventiv auf Teile des Gesichts topisch aufgetragen wird, welche von Fieberbläschen betroffen sein können.

2. Zusammensetzung zur Verwendung nach Anspruch 1, wobei die Zusammensetzung von dem Moment an auf das Gesicht aufgetragen wird, in welchem der Benutzer ein erhöhtes Risiko zur Bildung von Fieberbläschen hat.

3. Zusammensetzung zur Verwendung nach Anspruch 1, wobei die Zusammensetzung bis zu mehrere Tage aufgetragen wird, beispielsweise 1-5 Tage, nachdem das erhöhte Risiko verschwunden ist.

4. Zusammensetzung zur Verwendung nach Ansprüchen 2-3, wobei das erhöhte Risiko eine erhöhte Exposition mit UV Strahlung umfasst, und die Zusammensetzung in Intervallen von 2-4 Stunden während dieser erhöhten Exposition aufgetragen wird.

5. Zusammensetzung zur Verwendung nach Anspruch 1, wobei die Zusammensetzung für mehrere Tage auf das Gesicht aufgetragen wird, beispielsweise 1-5 Tage vor dem/den Tag/Tagen an welchem/welchen der Benutzer mit Sicherheit keine Fieberbläschen haben möchte.

6. Zusammensetzung zur Verwendung nach Anspruch 5, wobei die Zusammensetzung bis zu und inklusive des/der Tages/Tage aufgetragen wird, an welchem der Benutzer mit Sicherheit keine Fieberbläschen haben möchte.

7. Zusammensetzung zur Verwendung nach einem der Ansprüche 1-6, wobei das Zinksalz Zinksulfat ist.

8. Zusammensetzung zur Verwendung nach einem der Ansprüche 1-7, wobei die Zusammensetzung, in Bezug auf die Zink-Ionen, eine Menge des Zinksalzes von nicht mehr als 0,5% (Gew./Gew.) umfasst.

9. Zusammensetzung zur Verwendung nach einem der Ansprüche 1-8, wobei die Zusammensetzung zwischen 8-12% (Gew./Gew.), beispielsweise 10% (Gew./Gew.), Zinkoxid und/oder Titandioxid umfasst.

10. Zusammensetzung zur Verwendung nach einem der Ansprüche 1-9, in der Form einer Creme oder eines Gels.

11. Zusammensetzung zur Verwendung nach einem der Ansprüche 1-10, welche ferner Wasser in einer Menge von 50-60% (Gew./Gew.) beinhaltet, bevorzugt 52-58% (Gew./Gew.), am bevorzugtesten 55% (Gew./Gew.), und wobei das Additiv eine Öl/Fett-artige Substanz ist und die Wasserphase und die Öl/Fett-Phase eine Öl/Fett-in-Wasser-Emulsion bilden.

12. Zusammensetzung zur Verwendung nach Anspruch 11, wobei die Emulsion eine stabile Emulsion ist.

13. Zusammensetzung zur Verwendung nach einem der Ansprüche 1-12, wobei das Additiv so ausgewählt ist, dass die Zusammensetzung wasserbeständig ist.

14. Zusammensetzung zur Verwendung nach einem der Ansprüche 1-13, mit einem Lichtschutzfaktor (LSF) von mindestens 12, bevorzugt mindestens 15, am bevorzugtesten mindestens 20.

## Revendications

1. Composition pour une utilisation dans le traitement préventif de boutons de fièvre, y compris des boutons de fièvre récurrents, dans laquelle la composition comprend :
• 4 à 20 % (p/p) d'oxyde de zinc et/ou de dioxyde de titane ;
• en ce qui concerne les ions zinc, 0,1 à 2,0 % (p/p) d'un sel de zinc hydrosoluble ayant une action antivirale, ledit sel de zinc étant choisi parmi le groupe constitué de sulfate de zinc, chlorure de zinc, acétate de zinc, citrate de zinc, nitrate de zinc, tartrate de zinc, maléate de zinc, lactate de zinc, acétate aminé de zinc, aspartate de zinc, glutamate de zinc, propionate de zinc, gluconate de zinc, butyrate de zinc, formiate de zinc, glycéride de zinc, glycolate de zinc, et leurs hydrates ; et
• un additif adapté à une application topique ;
dans laquelle la composition est appliquée topiquement de manière préventive sur des parties du visage susceptibles d'être affectées par des boutons de fièvre.

2. Composition pour l'utilisation selon la revendication 1, dans laquelle la composition est appliquée sur le visage à partir du moment où l'utilisateur présente un risque accru de développer des boutons de fièvre.

3. Composition pour l'utilisation selon la revendication 2, dans laquelle la composition est appliquée jusqu'à plusieurs jours, tels que 1 à 5 jours, après la disparition du risque accru.

4. Composition pour l'utilisation selon les revendications 2 à 3, dans laquelle le risque accru comprend une exposition accrue à un rayonnement UV, et la composition est appliquée à des intervalles de 2 à 4 heures pendant cette exposition accrue.

5. Composition pour l'utilisation selon la revendication 1, dans laquelle la composition est appliquée sur le visage pendant plusieurs jours, tels que 1 à 5 jours, avant le ou les jours où l'utilisateur souhaite éviter avec certitude des boutons de fièvre.

6. Composition pour l'utilisation selon la revendication 5, dans laquelle la composition est appliquée jusqu'au(x) jour(s) choisi(s) inclus où l'utilisateur souhaite éviter avec certitude des boutons de fièvre.

7. Composition pour l'utilisation selon l'une quelconque des revendications 1 à 6, dans laquelle le sel de zinc est du sulfate de zinc.

8. Composition pour l'utilisation selon l'une quelconque des revendications 1 à 7, dans laquelle la composition en ce qui concerne les ions zinc comprend une quantité du sel de zinc non supérieure à 0,5 % (p/p).

9. Composition pour l'utilisation selon l'une quelconque des revendications 1 à 8, dans laquelle la composition comprend entre 8 et 12 % (p/p), tel que 10 % (p/p) d'oxyde de zinc et/ou de dioxyde de titane.

10. Composition pour l'utilisation selon l'une quelconque des revendications 1 à 9, sous la forme d'une crème ou d'un gel.

11. Composition pour l'utilisation selon l'une quelconque des revendications 1 à 10, qui contient en outre de l'eau selon une quantité de 50 à 60 % (p/p), de préférence de 52 à 58 % (p/p), de manière la plus préférée de 55 % (p/p), et dans laquelle l'additif est une substance de type huile/graisse et la phase aqueuse et la phase huile/graisse forment une émulsion huile/graisse dans eau.

12. Composition pour l'utilisation selon la revendication 11, dans laquelle l'émulsion est une émulsion stable.

13. Composition pour l'utilisation selon l'une quelconque des revendications 1 à 12, dans laquelle l'additif est choisi de telle sorte que la composition est résistante à l'eau.

14. Composition pour l'utilisation selon l'une quelconque des revendications 1 à 13, avec un facteur de protection solaire (FPS) d'au moins 12, de préférence d'au moins 15, et de manière la plus préférée d'au moins 20.
